# EUROPEAN PATENT SPECIFICATION

(11) **EP 1 222 294 B1**
(45) Date of publication and mention of the grant of the patent: **07.12.2005**
(21) Application number: 00967453.2
(22) Date of filing: 02.10.2000
(51) Int. Cl.: C12N 15/82, C12N 15/29

(54) **PROMOTER FOR REGULATING EXPRESSION OF FOREIGN GENES**
PROMOTOR ZUR REGULIERUNG VON FREMDGENEXPRESSION
PROMOTEUR DESTINE A REGULER L'EXPRESSION DE GENES ETRANGERS

(30) Priority: 04.10.1999 US 157129 P
(43) Date of publication of application: 17.07.2002
(73) Proprietor: Medicago Inc., Sainte-Foy, Québec G1K 7P4 (CA)
(72) Inventor: Vezina, Louis-Philippe, Neuville, Québec G0A 2R0 (CA); D'Aoust, Marc-André, Québec, Québec G1S 2W9 (CA)
(74) Representative: Guerre, Dominique
(86) International application number: PCT/CA2000/001144
(87) International publication number: WO 2001/025455

(56) References cited:
- WO-A-00/56906
- KENG-HOCK PWEE ET AL: "THE PEA PLASTOCYANIN PROMOTER DIRECTS CELL-SPECIFIC BUT NOT FULL LIGHT-REGULATED EXPRESSION IN TRANSGENIC TOBACCO PLANTS" PLANT JOURNAL,GB,BLACKWELL SCIENTIFIC PUBLICATIONS, OXFORD, vol. 3, no. 3, 1993, pages 437-449, XP002032746 ISSN: 0960-7412
- LAST D I ET AL: "PLASTOCYANIN IS ENCODED BY A SINGLE-COPY GENE IN THE PEA HAPLOID GENOME" PLANT MOLECULAR BIOLOGY,NL,NIJHOFF PUBLISHERS, DORDRECHT, vol. 12, 1989, pages 655-666, XP002032748 ISSN: 0167-4412

## Description

### BACKGROUND OF THE INVENTION

### (a) Field of the Invention

The invention relates to a promoter for regulating expression of foreign genes in a transgenic organism, more specifically in a leaf-specific manner in transgenic plants.

### (b) Description of Prior Art

Genetic transformation of microbes have been used for more than 15 years to produce useful recombinant molecules, and applications in the pharmaceutical, cosmaceutical and dermaceutical industries are being currently exploited. This technology has expanded from microbes to plants and animals in the last ten years with the development of techniques required to adapt this general concept to complex eukaryotic organisms. Basically a gene encoding for a protein of interest or a gene encoding for an enzyme responsible for a modification of a metabolic pathway that leads to a molecule of interest, is linked in an appropriate fashion to cis-and trans-acting regulatory sequences, and transferred to a target cell where it is incorporated in the molecular machinery (in a transitory or stable fashion). The transgenic cell, or a tissue or organism regenerated from the transgenic cell will then perform transcription and translation of the transgene and therefore be enabled to accumulate the protein of interest or to perform the new metabolic reaction through the activity of the enzyme of interest.

The emerging industry of molecular farming is one of the most promising industry of the coming century. Its promise is to provide safe and renewable molecule factories for the industry. Among the applications that are currently developed are the production of low-cost monoclonal antibodies for therapeutic and diagnostic uses, the production of unlimited amounts of hormones, cytokines and other bio-active molecules for the treatment of chronicle or lethal diseases, the production of bio-safe substitutes for various blood components, the production of unlimited amounts of processing enzymes for the food and pulp industry, the production of low-cost enzymes for waste treatments, and the production of safe bio-active molecules for the cosmetic industry.

Limitations to the application of this technology has often come from the inability of transgenic organisms to accumulate adequate amounts of the recombinant product, as a result of low transcription rates, improper splicing of the messenger, instability of the foreign mRNA, poor translation rates, hyper-susceptibility of the recombinant protein to the action of endogenous proteases or hyper-susceptibility of the recombinant organism to the foreign protein which result in improper and limited growth or in the worst cases, in strong deleterious effects to the host organism. Inadequacy of production level has a direct impact on the development of applications when profit margins are narrow, or when treatment and/or disposal of residual matter causes bio-safety or environmental problems. Improvement of the accumulation level of the desired recombinant product thus appears to be one critical factor that warrants commercialization of many applications of molecular farming.

Photosynthesis is a metabolic reaction of paramount importance in the living world. It is performed by most land plants and algae, and by some bacteria. This overall reaction involves a complex assembly of electron transfer proteins spatially arranged within the thylakoid membrane system located in the chloroplast of leaf cells. This electron transport chain is coupled at one end with the photosynthetic antennae, which comprise a variety of macro-molecules, including one molecule common to all photosynthetic organism, chlorophyll, and at the other end, to the enzymes involved in NADPH and ATP synthesis, and to the Calvin cycle, involved in coupling the release of energy from NADPH and ATP with the fixation of gaseous carbon dioxide into organic molecules. Among the proteins involved in the overall photosynthetic reaction, one, Ribulose biphosphate carboxylase (Rubisco), is the most abundant protein on earth.

Photosynthesis is thus what leaf cells are dedicated to perform, and there is an obvious interest to use promoters of genes involved in such prominent tissue-specific metabolic activity when building strong leaf-specific expression cassettes for applications in plant biotechnology.

Many of the peptidic constituents of the photosynthetic apparatus are encoded by genes present in the chloroplastic genome; as an example, the heavy subunit of Rubisco, which bears the catalytic sites for CO₂ fixation, is encoded by a chloroplastic gene. However, the small subunit of this enzyme is encoded by a nuclear gene, and thus the Rubisco holo-protein is made of subunits encoded by two different genomes. For obvious reasons, there has been a great interest in trying to use Rubisco promoters to control transcription of transgenes in leaves of transgenic plants. The promoter has been extensively characterized and its use in expression vectors is protected by United States Patent No. 4,962,028.

Keng-Hock Pwee *et al.* (The Plant Journal (1993) 3(3), 437-449) describes the use of a pea plastocyanin promoter sequence for expression of beta-glucuronidase (GUS) in transgenic tobacco plants. A series of 5' deletions of the pea plastocyanin gene promoter fused to the beta-glucuronidase reporter gene has been examined for expression in transgenic tobacco plants. Further modifications of this promoter sequence are not described.

It would be highly desirable to be provided with a promoter for regulating expression of foreign genes in a transgenic organism, more specifically in transgenic plants.

### SUMMARY OF THE INVENTION

One aim of the present invention is to provide with a promoter for regulating expression of foreign genes in a transgenic organism, more specifically in transgenic plants.

In accordance with one embodiment of the present invention, there is provided a promoter for regulating expression of foreign genes in transgenic organisms, which comprises a promoter having the identifying characteristics of a promoter having a sequence selected from the group consisting of sequences set forth in SEQ ID NOS:1 to 3 and functional fragments or derivatives thereof, wherein said promoter is adapted to be operationally located with respect to said foreign gene for expression of said gene.

The preferred promoter of the present invention has a sequence selected from the group consisting of sequences set forth in SEQ ID NOS:1 to 3.

Preferably, the organism is a plant.

Preferably, the promoter of the present invention may be modulated by the presence or absence of light.

The prefered plant is a dicot, a monocot or a gymnosperm.

In accordance with another embodiment of the present invention, there is provided a method of regulating expression of foreign genes in transgenic organisms, comprising the steps of:
a) preparing a transgenic organism using an expression construct consisting of at least a promoter of the present invention, and an ORF of a gene, wherein said promoter is operationally located with respect to said gene for expression of said gene.
   For the purpose of the present invention the following terms are defined below.

The expression "functional fragments or derivatives thereof" is intended to mean any derivative or fragment of sequences SEQ ID NOS:1-3 which allow for an equivalent level of expression of a foreign gene as the promoter of the present invention set forth in SEQ ID NOS:1-3.

### DETAILED DESCRIPTION OF THE INVENTION

Following is a detailed description of the method used to generate transgenic alfalfa lines that can be regulated in their expression of a reporter gene.

In this embodiment, a promoter having the sequence set forth in SEQ ID NOS:1-3 was then ligated to a reporter gene and a terminator, and this construct was inserted in suitable plant expression vectors for DNA bombardment onto alfalfa leaves and for *Agrobacterium* mediated DNA transfer as described by Desgagnés et al. (1995, *Plant Cell Tissue Organ Cult.* 42:129-140). These two DNA transfer methods were used to demonstrate that expression of the reporter gene can be modulated by light.

### Materials and Methods

### DNA sequencing

DNA sequencing was performed as described by Sanger et al (1977, *P.N.A.S. USA,* 74:5643-5647).

The resulting promoters of the present invention have the sequence as set forth in SEQ ID NOS: 1 to 3.

### Construction of expression cassettes and vectors

The cassettes for expression analysis using the GUS reporter gene were assembled as follows. A promoterless GUS cassette was digested from pBI101 with Hindlll and EcoRI, and was inserted into the HindIII and EcoRI sites of the pUC19 polycloning site. The resulting plasmid was named pBI2OI and was used for further constructs. SED ID NO:2 and SED ID NO:3 which are deletion fragments of SEQ ID NO:1 were transcriptionally and transitionally fused at the 5'terminus of the GUS reporter gene in pBI2OI and these were used for transitory expression studies using DNA bombardment. Upon identification of the adequate deletion fragment, it was subcloned into a binary plant expression vector such as pBIIOI (Clonetech). These recombinant plasmids were used for stable integration through *A*. *tumefaciens* infection as described below.

### Agrobacterium-mediated DNA transfer and regeneration of transgenic lines

The recombinant plasmids were introduced into *Agrobacterium tumefaciens* strain LBA4404 by electroporation as described in Khoudi et al (1999, Biotechnol. Bioeng., 64:135-143). Selected *Agrobacterium* strains were then co-cultivated with leaf disks from genotype C5-1 for 4 days in the absence of selection pressure (kanamycin). Following this incubation period, leaf disks were washed and pampered, and then allowed to form calli onto medium B5H. Calli were then transferred for 21 days on SH medium for embryo induction and for 28 days on BOi2Y for embryo development. Torpedo-shaped embryos were removed from Boi2Y and placed on MS medium for regeneration. Kanamycin was present in all cultivation medium except for co-cultivation and regeneration on MS. This method is described in length in Desgagnés et al (1995, *Plant Cell Tissue Organ Cult.* 42:129-140). Rooted plantlets were grown to maturity in the greenhouse.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. A promoter for regulating expression of a foreign gene in a transgenic plant, which comprises a nucleic acid sequence selected from the group consisting of sequences identified under SEQ ID NOS:1 to 3.

2. A construct comprising a promoter according to claim 1 and a foreign gene, wherein said promoter is adapted to be operationally located with respect to said foreign gene for expression of said gene.

3. Use of a promoter according to claim 1 for regulating the expression of a foreign gene in a transgenic plant.

4. The use of claim 3, wherein said plant is a dicot, a monocot or a gymnosperm.

5. The use of claim 3, wherein said promoter is modulated for transcriptional expression of said gene by presence or absence of light.

## Patentansprüche

1. Ein Promoter zur Regulierung der Expression eines fremden Gens in einer transgenen Pflanze, welcher eine Nukleinsäure-Sequenz umfasst, die gewählt ist aus der Gruppe bestehend aus Sequenzen, die unter Sequanzidentifikationsnummern 1 bis 3 identifiziert sind.

2. Ein Konstrukt, das einen Promoter gemäß Anspruch 1 und ein fremdes Gen umfasst, worin der Promoter angepasst ist, um in Bezug auf das frembe Gen operativ für die Expression des Gena angreordnet zu sein.

3. Verwendung eines Promoters gemäß Anspruch 1 zur Regulierung der Expression eines fremden Gens in einer transgenen Pflanze.

4. Verwendung von Anspruch 3, worin dia Pflanze ein Dikotyledon, ein Monokotyledon oder ein Gymnosperm ist.

5. Die Verwendung von Anspruch 3, worin der Promoter durch die Anwesenhelt oder Abwesenheit von Licht für die transkriptionelle Expression des Gens moduliert wird.

## Revendications

1. Un promoteur pour régler l'expression d'un gène étranger dans une plante transgénique, comprenant une séquence d'acide nucléique choisie dans le groupe constitué des séquences identifiées sous SEQ ID NOS :1 à 3.

2. Une construction comprenant un promoteur selon la revendication 1 et un gène étranger, **caractérisé en ce que** ledit promoteur est adapté pour être situé de facon opérationnelle par rapport audit gène étranger pour l'expression dudit gène.

3. Utilisation d'un promoteur selon la revendication 1 pour régler l'expression d'un gène étranger dans une plante transgénique.

4. Utilisation selon la revendication 3, **caractérisé en ce que** ladite plante est un dicot, un monocot ou un gymnosperme.

5. L'utilisation selon la revendication 3, **caractérisée en ce que** ledit promoteur est modulé pour l'expression transcriptionnelle dudit gène en présence ou en absence de lumière.


### SEQUENCE LISTING

<110> VÉZINA, Louis-Philippe
   D'AOUST, Marc-André
   MEDICAGO Inc.
<120> PROMOTER FOR REGULATING EXPRESSION OF FOREIGN GENES
<130> 14149-4"PCT"
<150> US 60/157,129
   <151> 1999-10-04
<160> 3
<170> FastSEQ for Windows Version 3.0
<210> 1
   <211> 1350
   <212> DNA
   <213> Artificial Sequence
<220>
   <223> Sequence to be used as a Promoter for regulating expression
<400> 1
   cgacggcccg ggctggtata tttatatgtt gtcaaataac tcaaaaacca taaaagttta      60
   agttagcaag tgtgtacatt tttatttgaa caaaaatatt cacctactac tgttataaat     120
   cattattaaa cattagagta aagaaatatg gatgataaga acaagagtag tgatattttg     180
   acaacaattt tgttgcaaca tttgagaaaa ttttgttgtt ctctcttttc attggtcaaa     240
   aacaatagag agagaaaaag gaagagggag aataaaaaca taatgtgagt atgagagaga     300
   aagttgtaca aaagttgtac caaaatagtt gtacaaatat cattgaggaa tttgacaaaa     360
   gctacacaaa taagggttaa ttgctgtaaa taaataagga tgacgcatta gagagatgta     420
   ccattagaga atttttggca agtcattaaa aagaaagaat aaattatttt taaaattaaa     480
   agttgagtca tttgattaaa catgtgatta tttaatgaat tgatgaaaga gttggattaa     540
   agttgtatta gtaattagaa tttggtgtca aatttaattt gacatttgat cttttcctat     600
   atattgcccc atagagtcag ttaactcatt tttatatttc atagatcaaa taagagaaat     660
   aacggtatat taatccctcc aaaaaaaaaa aacggtatat ttactaaaaa atctaagcca     720
   cgtaggagga taacatccaa tccaaccaat cacaacaatc ctgatgagat aacccacttt     780
   aagcccacgc actctgtggc acatctacat tatctaaatc acacattctt ccacacatct     840
   gagccacaca aaaaccaatc cacatcttta tcacccattc tataaaaaat cacactttgt     900
   gagtctacac tttgattccc ttcaaacaca tacaaagaga agagactaat taattaatta     960
   atcatcttga gagaaaatgg ccaccgttac ttccaccacc gttgctattc catcattcac    1020
   aggccttaag gcaaacgcaa gcaaagttaa tgccatagct aaggttccaa cttcaacttc    1080
   tcaattgcca aggctttgtg tcagagcttc cctcaaagac tttggagttg ctgctgttgc    1140
   cactgctgca agtgcattgt tagctagcaa tgcccttgca gttgaagtgt tgcttggtgc    1200
   tagtgatggg ggtttggctt ttgttccaaa caatttcaca gtgaacgctg gagacaccat    1260
   tacattcaag aacaatgctg gttttcctca caacgttatc ttcgatgaag acgagattcc    1320
   aagcggggtt gatgctgcaa tcgaattccc                                     1350
<210> 2
   <211> 971
   <212> DNA
   <213> Artificial Sequence
<220>
   <223> Sequence to be used as a Promoter for regulating expression
<400> 2
   cgggctggta tatttatatg ttgtcaaata actcaaaaac cataaaagtt taagttagca      60
   agtgtgtaca tttttatttg aacaaaaata ttcacctact actgttataa atcattatta     120
   aacattagag taaagaaata tggatgataa gaacaagagt agtgatattt tgacaacaat     180
   tttgttgcaa catttgagaa aattttgttg ttctctcttt tcattggtca aaaacaatag     240
   agagagaaaa aggaagaggg agaataaaaa cataatgtga gtatgagaga gaaagttgta     300
   caaaagttgt accaaaatag ttgtacaaat atcattgagg aatttgacaa aagctacaca     360
   aataagggtt aattgctgta aataaataag gatgacgcat tagagagatg taccattaga     420
   gaatttttgg caagtcatta aaaagaaaga ataaattatt tttaaaatta aaagttgagt     480
   catttgatta aacatgtgat tatttaatga attgatgaaa gagttggatt aaagttgtat     540
   tagtaattag aatttggtgt caaatttaat ttgacatttg atcttttcct atatattgcc     600
   ccatagagtc agttaactca tttttatatt tcatagatca aataagagaa ataacggtat     660
   attaatccct ccaaaaaaaa aaaacggtat atttactaaa aaatctaagc cacgtaggag     720
   gataacatcc aatccaacca atcacaacaa tcctgatgag ataacccact ttaagcccac     780
   gcactctgtg gcacatctac attatctaaa tcacacattc ttccacacat ctgagccaca     840
   caaaaaccaa tccacatctt tatcacccat tctataaaaa atcacacttt gtgagtctac     900
   actttgattc ccttcaaaca catacaaaga gaagagacta attaattaat taatcatctt     960
   gagagaaaat g                                                          971
<210> 3
   <211> 731
   <212> DNA
   <213> Artificial Sequence
<220>
   <223> Sequence to be used as a Promoter for regulating expression
<400> 3
   agagagaaaa aggaagaggg agaataaaaa cataatgtga gtatgagaga gaaagttgta    60
   caaaagttgt accaaaatag ttgtacaaat atcattgagg aatttgacaa aagctacaca   120
   aataagggtt aattgctgta aataaataag gatgacgcat tagagagatg taccattaga   180
   gaatttttgg caagtcatta aaaagaaaga ataaattatt tttaaaatta aaagttgagt   240
   catttgatta aacatgtgat tatttaatga attgatgaaa gagttggatt aaagttgtat   300
   tagtaattag aatttggtgt caaatttaat ttgacatttg atcttttcct atatattgcc   360
   ccatagagtc agttaactca tttttatatt tcatagatca aataagagaa ataacggtat   420
   attaatccct ccaaaaaaaa aaaacggtat atttactaaa aaatctaagc cacgtaggag   480
   gataacatcc aatccaacca atcacaacaa tcctgatgag ataacccact ttaagcccac   540
   gcactctgtg gcacatctac attatctaaa tcacacattc ttccacacat ctgagccaca   600
   caaaaaccaa tccacatctt tatcacccat tctataaaaa atcacacttt gtgagtctac   660
   actttgattc ccttcaaaca catacaaaga gaagagacta attaattaat taatcatctt   720
   gagagaaaat g                                                        731